Europäisches Patentamt

European Patent Office

Office européen des brevets

(19)

(11) **EP 0 749 752 B1**

(12) **FASCICULE DE BREVET EUROPEEN**

(45) Date de publication et mention
de la délivrance du brevet:
**08.05.2002 Bulletin 2002/19**

(51) Int Cl.7: **A61K 31/59**, A61K 31/20,
A61K 31/19, A61P 17/00

(21) Numéro de dépôt: **96401140.7**

(22) Date de dépôt: **28.05.1996**

(54) **Utilisation de ligands spécifiques des récepteurs RXRs**

Verwendung von spezifischen RXR-Rezeptorliganden

Use of specific RXR receptor ligands

(84) Etats contractants désignés:
**AT BE CH DE DK ES FI FR GB GR IE IT LI LU MC NL PT SE**

(30) Priorité: **19.06.1995 FR 9507300**

(43) Date de publication de la demande:
**27.12.1996 Bulletin 1996/52**

(73) Titulaire: **CENTRE INTERNATIONAL DE RECHERCHES DERMATOLOGIQUES GALDERMA (C.I.R.D. GALDERMA) 06560 Valbonne (FR)**

(72) Inventeurs:
• **Demarchez, Michel
  06620 Le Bar sur Loup (FR)**
• **Jomard, André
  06460 Saint Vallier de Thiey (FR)**

(74) Mandataire: **Andral, Christophe André Louis
L'OREAL-DPI
6 rue Bertrand Sincholle
92585 Clichy Cedex (FR)**

(56) Documents cités:
**WO-A-94/15901**      **WO-A-94/17796**

• **MOL. CELL. BIOL., vol. 15, no. 12, 1995, pages 6481-6487, XP000565384 B. ROY ET AL.: "Synergistic activation of retinoic acid-responsive genes and induction of ebryonal carcinoma cell differentiation by an RA receptor alpha , RARbeta or RARgamma-selective ligand in combination with a retinoid X receptor-specific ligand."**
• **J.BIOL. CHEM., vol. 267, no. 31, 1992, pages 22010-22013, XP000565364 E.D. ROSEN ET AL.: "Ligand-dependent synergy of thyroid hormone and retinoid receptors."**

**Description**

L'invention a trait à l'utilisation d'au moins un ligand spécifique des récepteurs RXRs dans la préparation d'une composition pharmaceutique à administrer par voie systémique.

On sait que l'acide rétinoïque *tout-trans* est un puissant modulateur (i.e. un inhibiteur ou, au contraire, un stimulateur, selon la nature des cellules traitées) de la différenciation et de la prolifération de nombreux types cellulaires normaux ou transformés. Par exemple, il inhibe la différenciation des cellules épithéliales, tels que les kératinocytes de l'épiderme. Il inhibe aussi la prolifération de nombreuses cellules transformées telles que les cellules de mélanomes.

On sait, d'une manière générale, que l'acide rétinoïque *tout-trans* (all-*trans* retinoic acid) agit sur la différenciation et la prolifération des cellules en interagissant avec des récepteurs nucléaires appelés RARs (Retinoic Acid Receptors) contenus dans le noyau cellulaire. Il existe, à ce jour, trois sous-types identifiés de récepteurs RAR appelés respectivement RAR-α, RAR-β et RAR-γ. Ces récepteurs, après fixation du ligand (i.e. de l'acide rétinoïque), interagissent avec la région promotrice de gènes régulés par l'acide rétinoïque au niveau d'éléments de réponses spécifiques. Pour se fixer sur les éléments de réponse, les RARs s'hétérodimèrisent avec un autre type de récepteurs connus sous le nom de RXRs. Le ligand naturel des RXRs est l'acide 9-*cis*-rétinoïque. Les RXRs sont considérés comme des "master regulatory proteins" car ils interagissent avec d'autres membres de la superfamille des récepteurs stéroïdiens/thyroïdiens pour former des hétérodimères, comme avec les RARs, tels que le récepteur de la vitamine D3 (VDR), le récepteur de la triiodothyroxine (TR) et les PPARs (Peroxisome Proliferator Activated Receptors). De plus, les RXRs peuvent interagir avec des éléments de réponse spécifiques sous forme d'homodimères.

De nombreux analogues structuraux synthétiques de l'acide rétinoïque *tout-trans* ou de l'acide 9-*cis*-rétinoïque, couramment dénommés "rétinoïdes", ont été décrits à ce jour dans la littérature. Certaines de ces molécules sont capables de se fixer et d'activer spécifiquement les RARs ou, au contraire, les RXRs. De plus, certains analogues peuvent se fixer et activer un sous-type de récepteur RAR (α, β ou γ) particulier. D'autres analogues, enfin, ne présentent aucune activité sélective particulière vis-à-vis de ces différents récepteurs. A cet égard, et par exemple, l'acide 9-*cis* rétinoïque active à la fois les RARs et les RXRs, sans sélectivité notable pour l'un ou l'autre de ces récepteurs (ligand non spécifique), alors que l'acide rétinoïque *tout-trans* active, quant à lui, sélectivement les RARs (ligand spécifique RARs), tous sous-types confondus. D'une manière générale et qualitativement, une substance donnée (ou ligand) est dite spécifique d'une famille de récepteurs donnée (respectivement vis-à-vis d'un récepteur particulier de cette famille) lorsque ladite substance présente une forte affinité pour l'ensemble des récepteurs de cette famille (respectivement pour le récepteur particulier de cette famille) et qu'elle présente par ailleurs une faible affinité pour tous les récepteurs de toute autre famille (respectivement pour tous les autres récepteurs, de cette même famille ou pas).

Du fait des activités mentionnées ci-dessus, il est également bien connu que l'acide rétinoïque, la vitamine D ou leurs analogues sont utilisés pour le traitement topique de diverses maladies dermatologiques ou dans le domaine cosmétique.

Cependant, leur utilisation peut entraîner des effets secondaires importants, tels que la tératogénicité et/ou l'irritation pour les ligands sélectifs des récepteurs RARs, et l'hypercalcémie pour la vitamine D ou ses analogues. Les ligands sélectifs pour les récepteurs RXRs sont connus, quant à eux, pour présenter une activité faible, voire nulle, tératogène, irritante et/ou hypercalcémiante.

La Demanderesse vient de découvrir, de manière tout à fait surprenante, que lorsque l'on applique, de manière topique, un ligand d'au moins un récepteur de la superfamille des récepteurs stéroïdiens/thyroïdiens, autre qu'un ligand spécifique des récepteurs RXRs, et pouvant s'hétérodimériser avec les RXRs, son activité peut être synergisée par l'administration par voie systémique (autre que topique : parentérale, entérale, dont la voie orale) d'au moins un ligand spécifique des récepteurs RXRs. Ce résultat est inattendu dans la mesure où un ligand spécifique des RXRs, lorsqu'il est utilisé seul et à des doses comparables, ne présente aucune ou substantiellement aucune activité. Il est d'autant plus surprenant d'observer cette synergie que les deux types de composés utilisés sont administrés par des voies différentes.

Ainsi, la présente invention a pour objet l'utilisation d'au moins un ligand spécifique des récepteurs RXRs dans la préparation d'une composition pharmaceutique à administrer par voie systémique et destinée à augmenter l'activité modulatrice de prolifération et/ou de différenciation cellulaire d'un ligand d'au moins un récepteur de la superfamille des récepteurs stéroïdiens/thyroïdiens, autre qu'un ligand spécifique des récepteurs RXRs, et pouvant s'hétérodimériser avec les RXRs, appliqué de manière topique.

La présente invention présente l'avantage de pouvoir diminuer les doses administrées topiquement des ligands d'au moins un récepteur de la superfamille des récepteurs stéroïdiens/thyroïdiens, autre qu'un ligand spécifique des récepteurs RXRs, et pouvant s'hétérodimériser avec les RXRs, tout en présentant la même activité et donc la même efficacité, mais en diminuant leurs effets secondaires, notamment tératogènes ou hypercalcémiant de certains de ces ligands, tels que respectivement les ligands spécifiques des récepteurs RARs ou du VDR.

D'autres caractéristiques, aspects, objets et avantages de l'invention apparaîtront encore plus clairement à la lecture de la description qui va suivre, ainsi que des divers exemples concrets, mais nullement limitatifs, destinés à l'illustrer.

Le ligand d'au moins un récepteur de la superfamille des récepteurs stéroïdiens/thyroïdiens, autre qu'un ligand spécifique des récepteurs RXRs, et pouvant s'hétérodimériser avec les RXRs, est de préférence choisi parmi les ligands des RARs (Retinoic Acid Receptors), du VDR (Vitamine D3 Receptor), des PPARs (Peroxisome Proliferator Activated Receptors) ou du TR (récepteur de la triiodothyroxine). De manière encore plus préférentielle, ce ligand est choisi parmi les ligands des RARs et les ligands du VDR.

De préférence, le ligand d'au moins un récepteur de la superfamille des récepteurs stéroïdiens/thyroïdiens, autre qu'un ligand spécifique des récepteurs RXRs, et pouvant s'hétérodimériser avec les RXRs, est spécifique de l'un de ces récepteurs. La spécificité de ce ligand est déterminée selon les méthodes indiquées ci-dessous.

Le caractère spécifique ou non d'une substance donnée vis-à-vis d'un ou plusieurs récepteurs nucléaires ou cytosoliques donnés peut être déterminé au moyen de tests classiques pour l'homme de l'art. Ces tests sont notamment décrits dans les références suivantes : (1) "Selective Synthetic Ligands for Nuclear Retinoic Acid Receptor Subtypes" *in* RETINOIDS, Progress in Research and Clinical Applications, Chapitre 19 (pp 261-267), Marcel Dekker Inc, édité par Maria A. Livrea et Lester Packer ; (2) "Synthetic Retinoids : Receptor Selectivity and Biological Activity" *in* Pharmacol Skin, Basel, Karger, 1993, Volume. 5, pp 117-127 ; (3) "Selective Synthetic Ligands for Human Nuclear Retinoic Acid Receptors" *in* Skin Pharmacology, 1992, Vol. 5, pp 57-65 ; (4) "Identification of Synthetic Retinoids with Selectivity for Human Nuclear Retinoic Acid Receptor-γ" *in* Biochemical and Biophysical Research Communications, Vol. 186, N° 2, Juillet 1992, pp 977-983 ; (5) "Selective High Affinity RAR-α or RAR-β Retinoic Acid Receptor Ligands" *in* Mol. Pharmacol., Vol 40, pp 556-562. Quantitavement, on admet de manière générale que toute substance qui, à l'égard d'un premier recepteur donné, présente une constante de dissociation (Kd) d'au moins 10 fois inférieur, et de préférence d'au moins 15 fois inférieur, au Kd qu'elle présente à l'égard d'un deuxième récepteur donné, peut être qualifiée de substance spécifique de ce premier récepteur par rapport à ce deuxième récepteur.

A titre d'exemples de ligands spécifiques de RARs utilisables dans le cadre de la présente invention, on peut notamment citer :

- l'acide rétinoïque *tout-trans*,
- l'acide 2-(5,6,7,8-tetrahydro-5,5,8,8-tetraméthyl-2-naphtyl)-6-benzo[b]thiophen carboxylique,
- l'acide 4-[(5,6,7,8-tétrahydro-5,5,8,8-tétraméthyl-2-naphtyl) carboxamido] benzoïque,
- l'acide 4-[(5,6,7,8-tétrahydro-5,5,8,8-tétraméthyl-2-naphtyl) carbamoyl] benzoïque,

Comme exemples de composés spécifiques du récepteur VDR, on peut citer les dérivés de la vitamine D suivants :

- 1α,25-dihydroxyvitamine D3,
- 1α-hydroxyvitamine D3,
- 25-hydroxyvitamine D3,
- 1α,25-dihydroxyvitamine D2,
- 1α,24-dihydroxyvitamine D2.

Parmi les ligands spécifiques des récepteurs PPARs, on peut notamment citer l'acide bromopalmitique et ses analogues.

Enfin, comme exemples de ligands spécifiques des RXRs convenant bien à l'invention, on peut ici citer plus particulièrement :

- l'acide 4-[(5,6,7,8-tétrahydro-5,5,8,8-tétraméthyl-2-naphtyl)carbonyléthylèneacétal]benzoïque,
- l'acide (E)-2-[2-(3,5,5,8,8-pentaméthyl-5,6,7,8-tétrahydro-2-naphtyl)-1-propényl]-4-thiophènecarboxylique,
- l'acide 4-[(3,5,5,8,8-pentaméthyl-5,6,7,8-tétrahydro-2-naphtyl)carbonyl] benzoïque.

Dans ce qui suit ou ce qui précède, on entend par voie topique, toute technique d'administration d'un produit par application directe de ce dernier sur une partie superficielle (ou externe) du corps, telle que la peau, le cuir chevelu, les ongles ou les muqueuses, et par voie systémique, toute technique d'administration d'un produit par une voie autre que topique, par exemple entérale et/ou parentérale. Dans le cas de la voie systémique, on préfère utiliser la voie orale.

Par voie entérale, et plus particulièrement orale, les compositions contenant le ou les ligand spécifique des RXRs peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, ces compositions peuvent se présenter sous forme de solutions ou de suspensions pour perfusion ou pour injection.

Le ou les ligands spécifiques des RXRs conformes à l'invention sont généralement administrés à des doses journalières d'environ 0,01 mg/kg à 100 mg/kg en poids corporel, et ceci à raison de 1 à 3 prises/jour, de préférence de 10 à 50 mg/kg.

Par voie topique, les compositions contenant le ou les ligands d'au moins un récepteur de la superfamille des récepteurs stéroïdiens/thyroïdiens, autre qu'un ligand spécifique des récepteurs RXRs, et pouvant s'hétérodimériser avec les RXRs, qui sont donc plus particulièrement destinées au traitement de la peau ou des muqueuses, peuvent se présenter sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée des actifs. Ces compositions par voie topique peuvent par ailleurs se présenter soit sous forme anhydre, soit sous une forme aqueuse, selon l'indication clinique.

Les compositions à usage topique conformes à l'invention contiennent le ou les ligands d'au moins un récepteur de la superfamille des récepteurs stéroïdiens/thyroïdiens, autre qu'un ligand spécifique des récepteurs RXRs, et pouvant s'hétérodimériser avec les RXRs, à une concentration généralement comprise entre 0,001 % et 10 % en poids, de préférence entre 0,01 et 1 % en poids, par rapport au poids total de la composition.

Les compositions destinées à un usage topique contenant le ou les ligands d'au moins un récepteur de la superfamille des récepteurs stéroïdiens/thyroïdiens, autre qu'un ligand spécifique des récepteurs RXRs, et pouvant s'hétérodimériser avec les RXRs, ou les compositions destinées à un usage systémique contenant le ou les ligands spécifiques des RXRs peuvent bien entendu en outre contenir des additifs inertes ou même pharmacodynamiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des agents antifongiques tels que le kétoconazole ou les polyméthylène-4,5 isothiazolidones-3; des antibactériens, des caroténoïdes et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'a-tocophérol, le butylhydroxyanisole ou le butylhydroxytoluène.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

L'activité modulatrice de prolifération et/ou de différenciation cellulaire des ligands d'au moins un récepteur de la superfamille des récepteurs stéroïdiens/thyroïdiens, autre qu'un ligand spécifique des récepteurs RXRs, et pouvant s'hétérodimériser avec les RXRs, leur permet donc d'être utilisés dans les domaines de traitement suivants :

1) Pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle.

2) Pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen cutané ou muqueux (buccal).

3) Pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; les composés peuvent également être utilisés dans certaines affections inflammatoires ne présentant pas de trouble de la kératinisation.

4) Pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires.

5) Pour traiter d'autres désordres dermatologiques tels que les dermatoses bulleuses et les maladies du collagène.

6) Pour traiter les cornéopathies.

7) Pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique.

8) Pour prévenir ou guérir les stigmates de l'atrophie épidermique et/ou dermique induite par les corticostéroïdes locaux ou systémiques, ou tout autre forme d'atrophie cutanée.

9) Pour prévenir ou traiter les troubles de la cicatrisation ou pour prévenir ou réparer les vergetures ou encore pour améliorer la cicatrisation.

10) Pour lutter contre les troubles de la fonction sébacée tels que l'hyperséborrhée de l'acné ou la séborrhée simple.

11) Dans le traitement ou la prévention des états cancéreux ou précancéreux susceptibles d'être traités par voie topique.

12) Dans le traitement d'affections inflammatoires telles que l'arthrite, susceptibles d'être traitées par voie topique.

13) Dans le traitement de toute affection d'origine virale au niveau cutané susceptibles d'être traitées par voie topique.

14) Dans la prévention ou le traitement de l'alopécie.

15) Dans le traitement d'affections dermatologiques à composante immunologique susceptibles d'être traitées par voie topique.

17) Dans le traitement ou la prévention de la cellulite.

18) Dans le traitement des mycoses, telles que l'onychomycose.

Les ligands d'au moins un récepteur de la superfamille des récepteurs stéroïdiens/thyroïdiens, autre qu'un ligand spécifique des récepteurs RXRs, et pouvant s'hétérodimériser avec les RXRs, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches, pour prévenir et/ou lutter contre le vieillissement photo-induit ou chronologique.

Ainsi, la présente invention a également pour objet un procédé cosmétique pour augmenter l'activité modulatrice de prolifération et/ou de différenciation cellulaire d'un ligand d'au moins un récepteur de la superfamille des récepteurs stéroïdiens/thyroïdiens, autre qu'un ligand spécifique des récepteurs RXRs, et pouvant s'hétérodimériser avec les RXRs, appliqué de manière topique, caractérisé en ce que l'on utilise, par voie systémique, au moins un ligand spécifique des récepteurs RXRs.

Dans le domaine cosmétique, comme dans le domaine pharmaceutique, les ligands d'au moins un récepteur de la superfamille des récepteurs stéroïdiens/thyroïdiens, autre qu'un ligand spécifique des récepteurs RXRs, et pouvant s'hétérodimériser avec les RXRs, ou les ligands spécifiques des RXRs peuvent être employés en combinaison avec d'autres rétinoïdes, avec d'autres vitamines D ou leurs dérivés, avec des corticostéroïdes, ou encore en association avec des anti-radicaux libres, avec des hydroxy ou céto acides ou leurs dérivés, ou encore avec des bloqueurs de canaux ioniques.

On va maintenant donner plusieurs exemples destinés d'une part à démontrer les effets attachés à la présente invention, et, d'autre part, à illustrer diverses formulations concrètes conformes à l'invention.

EXEMPLE 1

Cet exemple a pour but de mettre en évidence l'effet de synergie apporté par un ligand spécifique de RXRs donné par voie orale *in vivo* sur l'activité d'un ligand spécifique RARs appliqué par voie topique sur l'oreille de souris.

Il a en effet été montré que l'acide rétinoïque, qui est utilisé pour le traitement topique de diverses maladies dermatologiques, en administration unique par voie topique sur l'oreille de la souris induit une prolifération de l'épiderme (Arch. Dermatol. Res. 1992, 284:418-423), un érythème et un oedème de cette oreille, ces réponses étant dépendantes de la dose administrée. Ces signes cliniques sont également classiquement observés lors de l'application topique de l'acide rétinoïque chez l'homme.

De plus, il a été montré que ces effets peuvent être inhibés spécifiquement par un antagoniste des récepteurs RARs, ce qui est en faveur d'un lien mécanistique entre l'interaction du ligand avec ses récepteurs et les effets cliniques induits.

Le test utilisé pour évaluer cet effet de synergie est donc celui de l'oedème de l'oreille de souris induit par application topique de l'acide 2-(5,6,7,8-tetrahydro-5,5,8,8-tetraméthyl-2-naphtyl)-6-benzo[b]thiophen carboxylique (analogue de l'acide rétinoïque) à 0,003% en poids par volume. Selon ce modèle, une application topique de l'acide 2-(5,6,7,8-tetrahydro-5,5,8,8-tetraméthyl-2naphtyl)-6-benzo[b]thiophen carboxylique sur l'oreille provoque une inflammation qui se caractérise par une augmentation de l'épaisseur de l'oreille, ce dernier devenant maximal au bout de 5 jours après l'application. Cette réponse peut donc être quantifiée par une mesure de l'épaisseur de l'oreille par un oditest.

Le protocole opératoire exact est le suivant : 10 souris sont tout d'abord traitées avec l'acide 2-(5,6,7,8-tetrahydro-5,5,8,8-tetraméthyl-2-naphtyl)-6-benzo[b]thiophen carboxylique (composé A), en procédant sur l'une de leur oreille à une application topique à un temps t=0 avec 20 μl d'une solution d'acétone comprenant 0,003 % en poids par volume d'acide carboxylique 2-(5,6,7,8-tetrahydro-5,5,8,8-tetraméthyl-2naphtyl)-6-benzo[b]thiophen. On fait absorber par voie orale à 5 (= groupe 2) sur 10 des souris ainsi traitées de l'acide 4-[(3,5,5,8,8-pentaméthyl-5,6,7,8-tétrahydro-2-naphtyl)carbonyl] benzoïque (composé B) dans de l'huile de type cremophor (EL 25%) à partir de t=0 et ceci une fois par jour pendant 11 jours. Les 5 souris n'ayant pas absorbé le composé B constituent le groupe 1. La réponse oedémateuse est quantifiée par une mesure de l'épaisseur de l'oreille à t = ou 6 jours. Les résultats sont ensuite exprimés en % de l'effet de synergie calculé de la façon suivante :

$$\frac{\text{épaisseur oreille souris (Groupe 2) - épaisseur oreille souris (Groupe 1)}}{\text{épaisseur oreille souris (Groupe 1)}} \times 100$$

Les résultats obtenus sont rassemblés dans le tableau 1 suivant.

tableau 1

| voie topique | dose (%PN) | voie orale | dose (mg/kg) | effet de synergie (%) |
|---|---|---|---|---|
| composé A | 0,003 | composé B | 0 | 0 |
| composé A | 0,003 | composé B | 10 | 73 |
| composé A | 0,003 | composé B | 30 | 118 |
| P/V signifie poids par volume. | | | | |

De plus, l'application de manière analogue par voie orale d'un ligand spécifique des RXRs seul, tel que le composé B, n'induit pas de réponse sur la souris.

Ainsi, on démontre clairement grâce à ce test que l'association d'un ligand d'au moins un récepteur de la superfamille des récepteurs stéroïdiens/thyroïdiens, autre qu'un ligand spécifique des récepteurs RXRs, et pouvant s'hétérodimériser avec les RXRs avec un ligand spécifique RXRs augmente de manière importante la réponse de l'oreille de souris comparativement à la réponse induite par une application topique unique d'un ligand d'au moins un récepteur de la superfamille des récepteurs stéroïdiens/thyroïdiens, autre qu'un ligand spécifique des récepteurs RXRs, et pouvant s'hétérodimériser avec les RXRs.

EXEMPLE 2

Dans cet exemple, on a illustré diverses formulations concrètes (les composés A et B sont ceux définis ci-avant dans l'exemple 1). Les formulations comprenant le composé A sont destinées à une administration par voie topique et les formulations comprenant le composé B sont destinées à une administration par voie orale, les deux formulations devant être prises en association lors d'un traitement. Ainsi, les formulations comprenant le composé B peuvent être administrées oralement avant, pendant ou après l'application topique des formulations comprenant le composé A.

A- <u>VOIE ORALE</u>

(a) Comprimé de 0,2 g

- Composé B     0,002 g
- Amidon     0,113 g
- Phosphate bicalcique     0,020 g
- Silice     0,020 g
- Lactose     0,030 g
- Talc     0,010 g
- Stéarate de magnésium     0,005 g

(b) Suspension buvable en ampoules de 10 ml

- Composé B     0,1 g
- Glycérine     1,000g
- Sorbitol à 70%     1,000g
- Saccharinate de sodium     0,010 g
- Parahydroxybenzoate de méthyle     0,080 g
- Arome     qs
- Eau purifiée     qsp     10 ml

B- <u>VOIE TOPIQUE</u>

(a) Onguent

- Composé A     0,2 g
- Myristate d'isopropyle     81,520 g
- Huile de vaseline fluide     9,100 g
- Silice ("Aérosil 200" vendue par DEGUSSA)     9,180 g

(b) Crème Eau-dans-Huile non ionique

- Composé A     0,100 g
- Mélange d'alcools de lanoline émulsifs, de cires et d'huiles ("Eucerine anhydre" vendu par BDF)     39,900 g
- Parahydroxybenzoate de méthyle     0,075 g
- Parahydroxybenzoate de propyle     0,075 g
- Eau déminéralisée stérile     qsp     100 g

(c) Lotion

- Composé A     0,200 g
- Polyéthylène glycol (PEG 400)     69,800 g
- Ethanol à 95%     30,000 g

(d) Onguent hydrophobe

- Composé A          0,600 g
- Myristate d'isopropyle          36,400 g
- Huile de silicone ("Rhodorsil 47 V 300" vendu par RHONE-POULENC)          36,400 g
- Cire d'abeille          13,600 g
- Huile de silicone ("Abil 300.000 cst" vendu par GOLDSCHMIDT)          qsp          100g

(e) Crème Huile-dans-Eau non ionique

- Composé A          1,000 g
- Alcool cétylique          4,000 g
- Monostéarate de glycérole          2,500 g
- Stéarate de PEG 50          2,500 g
- Beurre de karité          9,200 g
- Propylène glycol          2,000 g
- Parahydroxybenzoate de méthyle          0,075 g
- Parahydroxybenzoate de propyle          0,075 g
- Eau déminéralisée stérile          qsp          100 g

**Revendications**

1. Utilisation d'au moins un ligand spécifique des récepteurs RXRs pour préparer une composition pharmaceutique à administrer par voie systémique et d'au moins un ligand d'au moins un récepteur de la superfamille des récepteurs stéroïdiens/thyroïdiens, autre qu'un ligand spécifique des récepteurs RXRs, et pouvant s'hétérodimériser avec les RXRs à l'activité modulatrice de prolifération et/ou de différenciation cellulaire pour préparer une composition à administrer par voie topique, lesdites compositions appliquées simultanément étant destinées:

   - au traitement des affections ou désordres dermatologiques, ou
   - au traitement des coméopathies, ou
   - à la réparation ou à la lutte contre le vieillissement de la peau ou à la réduction des pigmentations et des kératoses actiniques, ou au traitement de toutes pathologies associées au vieillissement chronologique ou actinique, ou
   - à la prévention ou à la guérison des stigmates de l'atrophie épidermique et/ou dermique, ou
   - à la prévention ou au traitement des troubles de la cicatrisation ou à la prévention ou à la réparation des vergetures, ou encore pour améliorer la cicatrisation, ou
   - à la lutte contre les troubles de la fonction sébacée, ou
   - au traitement ou à la prévention des états cancéreux ou précancéreux susceptibles d'être traités par voie topique, ou
   - au traitement d'affections inflammatoires susceptibles d'être traitées par voie topique, ou
   - au traitement de toute affection d'origine virale au niveau cutané susceptibles d'être traitées par voie topique, ou
   - à la prévention ou au traitement de l'alopécie, ou
   - au traitement d'affections dermatologiques à composante immunologique susceptibles d'être traitées par voie topique, ou

   - au traitement ou à la prévention de la cellulite, ou
   - au traitement de mycoses.

2. Utilisation selon l'une des revendications précédentes, **caractérisé en ce que** le ligand d'au moins un récepteur de la superfamille des récepteurs stéroïdiens/thyroïdiens, autre qu'un ligand spécifique des récepteurs RXRs, et pouvant s'hétérodimériser avec les RXRs, est choisi parmi les ligands des RARs, du VDR, des PPARs, du TR.

**3.** Utilisation selon la revendication précédente, **caractérisé en ce que** le ligand d'au moins un récepteur de la superfamille des récepteurs stéroïdiens/thyroïdiens, autre qu'un ligand spécifique des récepteurs RXRs, et pouvant s'hétérodimériser avec les RXRs, est choisi parmi les ligands des RARs et les ligands du VDR.

**4.** Utilisation selon l'une quelconque des revendications précédentes, **caractérisé en ce que** le ligand d'au moins un récepteur de la superfamille des récepteurs stéroïdiens/thyroïdiens, autre qu'un ligand spécifique des récepteurs RXRs, et pouvant s'hétérodimériser avec les RXRs, est spécifique de l'un de ces récepteurs.

**5.** Utilisation selon l'une quelconque des revendications précédentes, **caractérisé en ce que** le ligand spécifique des RARs est choisi parmi :

- l'acide rétinoïque *tout-trans*,
- l'acide 2-(5,6,7,8-tetrahydro-5,5,8,8-tetraméthyl-2-naphtyl)-6-benzo[b]thiophen carboxylique,
- l'acide 4-[(5,6,7,8-tétrahydro-5,5,8,8-tétraméthyl-2-naphtyl) carboxamido] benzoïque,
- l'acide 4-[(5,6,7,8-tétrahydro-5,5,8,8-tétraméthyl-2-naphtyl) carbamoyl] benzoïque.

**6.** Utilisation selon l'une quelconque des revendications précédentes, **caractérisé en ce que** le ligand spécifique des RXRs est choisi parmi :

- l'acide 4-[(5,6,7,8-tétrahydro-5,5,8,8-tétraméthyl-2-naphtyl)carbonyléthylèneacétal]benzoïque,
- l'acide (E)-2-[2-(3,5,5,8,8-pentaméthyl-5,6,7,8-tétrahydro-2-naphtyl)-1-propényl]-4-thiophènecarboxylique,
- l'acide 4-[(3,5,5,8,8-pentaméthyl-5,6,7,8-tétrahydro-2-naphtyl)carbonyl] benzoïque.

**7.** Utilisation selon l'une quelconque des revendications précédentes, **caractérisé en ce que** le ligand spécifique du récepteur VDR est choisi parmi :

- $1\alpha$,25-dihydroxyvitamine D3,
- $1\alpha$-hydroxyvitamine D3,
- 25-hydroxyvitamine D3,
- $1\alpha$,25-dihydroxyvitamine D2,
- $1\alpha$,24-dihydroxyvitamine D2.

**8.** Utilisation selon l'une quelconque des revendications précédentes, **caractérisé en ce que** la composition pharmaceutique comprenant au moins un ligand spécifique d'au moins un récepteur RXR est administrée par voie orale.

**9.** Utilisation selon l'une quelconque des revendications précédentes, **caractérisé en ce que** le ligand spécifique d'au moins un récepteur RXR est administré à des doses journalières d'environ 0,01 à 100 mg/kg en poids corporel.

**10.** Utilisation selon l'une quelconque des revendications précédentes, **caractérisé en ce que** le ligand d'au moins un récepteur de la superfamille des récepteurs stéroïdiens/thyroïdiens, autre qu'un ligand spécifique des récepteurs RXRs, et pouvant s'hétérodimériser avec les RXRs, est administré à une concentration généralement comprise entre 0,001 % et 10 % en poids, de préférence entre 0,01 et 1 % en poids, par rapport au poids total de la composition.

**11.** Procédé cosmétique pour augmenter l'activité modulatrice de prolifération et/ou de différenciation cellulaire d'un ligand d'au moins un récepteur de la superfamille des récepteurs stéroidiens/thyroïdiens, autre qu'un ligand spécifique des récepteurs RXRs, et pouvant s'hétérodimériser avec les RXRs, appliqué de manière topique, **caractérisé en ce que** l'on utilise, par voie systémique, au moins un ligand spécifique des récepteurs RXRs.

**Patentansprüche**

**1.** Verwendung mindestens eines spezifischen Liganden für die Rezeptoren RXR zur Herstellung einer pharmazeutischen Zusammensetzung, die auf systemischem Weg verabreicht werden soll, und mindestens eines Liganden für mindestens einen zur Bildung von Heterodimeren mit den RXRs befähigten Rezeptor aus der Qroßfamilie der Steroidhormon-/Thyroidhormon-Rezeptoren, der von einem spezifischen Liganden für die Rezeptoren RXR verschieden ist, mit modulatorischer Aktivität auf die Zellproliferation und/oder Zelldifferenzierung zur Herstellung einer Zusammensetzung, die auf topischem Weg verabreicht werden soll, wobei die Zusammensetzungen zusammen angewendet werden und vorgesehen sind für:

- die Behandlung von Hautkrankheiten oder Hautstömngen oder
- die Behandlung von Corneopathien oder
- die Behebung oder die Bekämpfung der Hautalterung oder die Verminderung der Pigmentierung und von Lichtkeratosen oder die Behandlung beliebiger Krankheiten, die mit der altersbedingten oder aktinischen Alterung verbunden sind, oder
- die Vorbeugung oder die Bekämpfung von Zeichen der epidermalen und/oder dermalen Atrophie oder
- die Vorbeugung oder die Behandlung von Störungen der Wundheilung oder die Vorbeugung oder die Behebung von atrophischen Hautstreifen oder auch die Verbesserung der Wundheilung oder
- die Bekämpfung von Störungen der Talgdrüsenfunktion oder
- die Behandlung oder die Vorbeugung von kanzerösen oder präkanzerösen Zuständen, die auf topischem Weg behandelt werden können, oder
- die Behandlung von entzündlichen Erkrankungen, die auf topischem Weg behandelt werden können, oder
- die Behandlung beliebiger Hautkrankheiten viralen Ursprungs, die auf topischem Weg behandelt werden können, oder
- die Vorbeugung oder die Behandlung von Alopezie oder
- die Behandlung von Hautkrankheiten mit immunologischer Komponente, die auf topischem Weg behandelt werden können, oder
- die Behandlung oder die Vorbeugung von Cellulitis oder
- die Behandlung von Mykosen.

2. Verwendung nach einem der vorhergehenden Ansprüche, **dadurch gekennzeichnet, dass** der Ligand für mindestens einen zur Bildung von Heterodimeren mit den RXRs befähigten Rezeptor aus der Großfamilie der Steroidhormon-/Thyroidhormon-Rezeptoren, der von einem spezifischen Liganden für die Rezeptoren RXR verschieden ist, unter den Liganden für die RARs, den VDR, die PPARs und den TR ausgewählt ist.

3. Verwendung nach dem vorhergehenden Anspruch, **dadurch gekennzeichnet, dass** der Ligand für mindestens einen zur Bildung von Heterodimeren mit den RXRs befähigten Rezeptor aus der Großfamilie der Steroidhormon-/Thyroidhormon-Rezeptoren, der von einem spezifischen Liganden für die Rezeptoren RXR verschieden ist, unter den Liganden für die RARs und den Liganden für den VDR ausgewählt ist.

4. Verwendung nach einem der vorhergehenden Ansprüche, **dadurch gekennzeichnet, dass** der Ligand für mindestens einen zur Bildung von Heterodimeren mit den RXRs befähigten Rezeptor aus der Großfamilie der Steroidhormon-/Thyroidhormon-Rezeptoren, der von einem spezifischen Liganden für die Rezeptoren RXR verschieden ist, für einen dieser Rezeptoren spezifisch ist.

5. Verwendung nach einem der vorhergehenden Ansprüche, **dadurch gekennzeichnet, dass** der für die RARs spezifische Ligand ausgewählt ist unter:

- *alltrans*-Retinsäure,
- 2-(5,6,7,8-Tetrahydro-5,5,8,8-tetramethyl-2-naphthyl)-6-benzo[*b*]-thiophen-carbonsäure,
- 4-[(5,6,7,8-Tetrahydro-5,5,8,8-tetramethyl-2-naphthyl)-carboxamido]-benzoesäure und
- 4-[(5,6,7,8-Tetrahydro-5,5,8,8-tetramethyl-2-naphthyl)-carbamoyl]-benzoesäure.

6. Verwendung nach einem der vorhergehenden Ansprüche, **dadurch gekennzeichnet, dass** der für die RXRs spezifische Ligand ausgewählt ist unter:

- 4-[(5,6,7,8-Tetrahydro-5,5,8,8-tetramethyl-2-naphthyl)-carbonylethylenacetal]-benzoesäure,
- (E)-2-[2-(3,5,5,8,8-Pentamethyl-5,6,7,8-tetrahydro-2-naphthyl)-1-propenyl]-4-thiophen-carbonsäure und
- 4-[(3,5,5,8,8-Pentamethyl-5,6,7,8-tetrahydro-2-naphthyl)-carbonyl]-benzoesäure.

7. Verwendung nach einem der vorhergehenden Ansprüche, **dadurch gekennzeichnet, dass** der spezifische Ligand für den Rezeptor VDR ausgewählt ist unter:

- $1\alpha$,25-Dihydroxyvitamin D3,
- $1\alpha$-Hydroxyvitamin D3,
- 25-Hydroxyvitamin D3,

- 1α,25-Dihydroxyvitamin D2 und
- 1α,24-Dihydroxyvitamin D2.

8. Verwendung nach einem der vorhergehenden Ansprüche, **dadurch gekennzeichnet, dass** die pharmazeutische Zusammensetzung, die mindestens einen spezifischen Liganden für mindestens einen Rezeptor RXR enthält, auf oralem Weg verabreicht wird.

9. Verwendung nach einem der vorhergehenden Ansprüche, **dadurch gekennzeichnet, dass** der spezifische Ligand für mindestens einen Rezeptor RXR mit einer täglichen Dosis von etwa 0,01 bis 100 mg pro kg Körpergewicht verabreicht wird.

10. Verwendung nach einem der vorhergehenden Ansprüche, **dadurch gekennzeichnet, dass** der Ligand für mindestens einen zur Bildung von Heterodimeren mit den RXRs befähigten Rezeptor aus der Großfamilie der Steroidhormon-/Thyroidhormon-Rezeptoren, der von einem spezifischen Liganden für die Rezeptoren RXR verschieden ist, in einer Konzentration verabreicht wird, die im allgemeinen im Bereich von 0,001 bis 10 Gew.-% und vorzugsweise von 0,01 bis 1 Gew.-%, bezogen auf das Gesamtgewicht der Zusammensetzung, liegt.

11. Kosmetisches Verfahren zur Erhöhung der modulatorischen Aktivität eines topisch angewendeten Liganden für mindestens einen zur Bildung von Heterodimeren mit den RXRs befähigten Rezeptor aus der Großfamilie der Steroidhormon-/Thyroidhormon-Rezeptoren, der von einem spezifischen Liganden für die Rezeptoren RXR verschieden ist, auf die Zellproliferation und/oder Zelldifferenzierung, **dadurch gekennzeichnet, dass** auf systemischem Weg mindestens ein für die Rezeptoren RXR spezifischer Ligand verwendet wird.

**Claims**

1. Use of at least one ligand which is specific for RXR receptors to prepare a pharmaceutical composition to be administered systemically and at least one ligand for at least one receptor of the superfamily of steroidal/thyroidal receptors, other than a ligand which is specific for the RXR receptors, and which can heterodimerize with the RXRs, having a cellular proliferation and/or differentiation modulatory activity to prepare a composition to be administered topically, the said compositions, which are applied simultaneously, being intended for:

   - the treatment of dermatological complaints or disorders, or
   - the treatment of corneopathies, or
   - repairing or combating ageing of the skin or reducing actinic keratoses and pigmentations, or for treating any pathologies associated with chronological or actinic ageing, or
   - the prevention or cure of stigmata of epidermal and/or dermal atrophy, or
   - the prevention or treatment of cicatrization disorders or the prevention or repair of stretchmarks, or alternatively for improving cicatrization, or
   - combating disorders of sebaceous functioning, or
   - the treatment or prevention of cancerous or precancerous states which can be treated topically, or
   - the treatment of inflammatory complaints which can be treated topically, or
   - the treatment of any skin complaint of viral origin which can be treated topically, or
   - the prevention or treatment of alopecia, or
   - the treatment of dermatological complaints having an immunological component which can be treated topically, or
   - the treatment or prevention of cellulitis, or
   - the treatment of mycoses.

2. Use according to the preceding claim, **characterized in that** the ligand for at least one receptor of the superfamily of steroidal/thyroidal receptors, other than a ligand which is specific for the RXR receptors, and which can heterodimerize with the RXRs, is chosen from ligands for the RARs, the VDR, the PPARs and the TR.

3. Use according to the preceding claim, **characterized in that** the ligand for at least one receptor of the superfamily of steroidal/thyroidal receptors, other than a ligand which is specific for the RXR receptors, and which can heterodimerize with the RXRs, is chosen from the ligands for the RARs and the ligands for the VDR.

4. Use according to any one of the preceding claims, **characterized in that** the ligand for at least one receptor of the superfamily of steroidal/thyroidal receptors, other than a ligand which is specific for the RXR receptors, and which can heterodimerize with the RXRs, is specific for one of these receptors.

5. Use according to any one of the preceding claims, **characterized in that** the ligand which is specific for the RARs is chosen from:

   - *all-trans*-retinoic acid,
   - 2-(5,6,7,8-tetrahydro-5,5,8,8-tetramethyl-2-naphthyl)-6-benzo[b]thiophenecarboxylic acid,
   - 4-[(5,6,7,8-tetrahydro-5,5,8,8-tetramethyl-2-naphthyl)carboxamido]benzoic acid,
   - 4-[(5,6,7,8-tetrahydro-5,5,8,8-tetramethyl-2-naphthyl)carbamoyl]benzoic acid.

6. Use according to any one of the preceding claims, **characterized in that** the ligand which is specific for the RXRs is chosen from:

   - 4-[(5,6,7,8-tetrahydro-5,5,8,8-tetramethyl-2-naphthyl)carbonylethyleneacetal]benzoic acid,
   - (E)-2-[2-(3,5,5,8,8-pentamethyl-5,6,7,8-tetrahydro-2-naphthyl)-1-propenyl]-4-thiophenecarboxylic acid,
   - 4-[(3,5,5,8,8-pentamethyl-5,6,7,8-tetrahydro-2-naphthyl)carbonyl]benzoic acid.

7. Use according to any one of the preceding claims, **characterized in that** the ligand which is specific for the VDR receptor is chosen from:

   - $1\alpha$,25-dihydroxyvitamin D3,
   - $1\alpha$-hydroxyvitamin D3,
   - 25-hydroxyvitamin D3,
   - $1\alpha$,25-dihydroxyvitamin D2,
   - $1\alpha$,24-dihydroxyvitamin D2.

8. Use according to any one of the preceding claims, **characterized in that** the pharmaceutical composition comprising at least one ligand which is specific for at least one RXR receptor is administered via the oral route.

9. Use according to any one of the preceding claims, **characterized in that** the ligand which is specific for at least one RXR receptor is administered at daily doses of approximately from 0.01 to 100 mg/kg of body weight.

10. Use according to any one of the preceding claims, **characterized in that** the ligand for at least one receptor of the superfamily of steroidal/thyroidal receptors, other than a ligand which is specific for the RXR receptors, and which can heterodimerize with the RXRs, is administered at a concentration generally of between 0.001 % and 10 % by weight, preferably of between 0.01 and 1 % by weight, relative to the total weight of the composition.

11. Cosmetic process for increasing the cellular proliferation and/or differentiation modulatory activity of a ligand for at least one receptor of the superfamily of steroidal/thyroidal receptors, other than a ligand which is specific for the RXR receptors, and which can heterodimerize with the RXRs, which is applied topically, **characterized in that** at least one ligand which is specific for the RXR receptors is used, via the systemic route.